# EUROPEAN PATENT SPECIFICATION

(11) **EP 2 229 191 B1**
(45) Date of publication and mention of the grant of the patent: **25.01.2012**
(21) Application number: 08867075.7
(22) Date of filing: 04.12.2008
(51) Int. Cl.: A61L 27/22, A61L 27/36, A61L 27/38, A61L 27/54

(54) **DECELLULARIZED OMENTUM MATRIX AND USES THEREOF**
DEZELLULARISIERTE OMENTUMMATRIX UND IHRE VERWENDUNG
EPIPLOON DECELLULARISE ET SON UTILISATION

(30) Priority: 19.12.2007 US 960010
(43) Date of publication of application: 22.09.2010
(73) Proprietor: ETHICON, INC., Somerville, NJ 08876-1515 (US)
(72) Inventor: YANG, Chunlin, Belle Mead New Jersey 08502 (US); JOHN, Thoppil Mathew, Easton Pennsylvania 18040 (US); GOSIEWSKA, Anna, Skillman New Jersey 08558 (US); BUENSUCESO, Charito S., New Jersey 08902 (US); COLTER, David C., Hamilton New Jersey 08610 (US); SEYDA, Agnieszka, Edison New Jersey 08817 (US); SHISSIAS, Raymond S., Iselin New Jersey 08830 (US)
(74) Representative: James, Anthony Christopher W.P.
(86) International application number: PCT/US2008/085450
(87) International publication number: WO 2009/085547

(56) References cited:
- US-A1- 2004 052 830
- US-A1- 2006 067 983
- HARA A ET AL: "Lipid extraction of tissues with a low-toxicity solvent" ANALYTICAL BIOCHEMISTRY, ACADEMIC PRESS INC, NEW YORK, vol. 90, no. 1, 1 October 1978 (1978-10-01), pages 420-426, XP024825165 ISSN: 0003-2697 [retrieved on 1978-10-01]

## Description

The invention relates generally to the field of biomatrix for tissue repair and regeneration. The invention concerns methods for extracting fat from omentum, processes for preparation of decellularized omentum and the application of decellularized omentum for tissue repair and tissue engineering.

### BACKGROUND OF THE INVENTION

Extracellular matrix (ECM) is an important structural component of connective tissues. ECM elaborated by cells creates microenvironments that these and other cells will respond to, by differentiating or maintaining their differentiated state. ECM provides a substrate for organization of cells which adhere to it.

Tissue based ECM biomaterial and devices are used for a variety of medical applications, such as heart valves, porcine SIS, human dermis and bovine pericardium. The application of decellularized tissues as tissue engineering scaffolds for regenerative medicine, however, is limited due to a lack of vascular tracks for angiogenesis, which is vital for tissue ingrowth and the viability and functionality of seeded cells. Tissue that is highly vascularized with the vascular tracks maintained during the decellularization process would be welcomed in the art.

US-A-2004052830 describes methods to decellularize cardiac valve or vessel tissue. The methods comprise treating tissue with a solution of bile acid to release the cells present in the tissue. The treated tissue is then rinsed in a phosphate buffer solution to remove the cells from the tissue matrix. Finally, the tissue is treated at room temperature for about 10 minutes in 40% alcohol to produce an antiviral effect and kill any remaining cells in the collagen structure.

A. Hara et al., in Analytical Biochemistry, vol. 90(1) pages 420-426 (1978) describe a tissue defatting process using a hexane:isopropanol solvent.

The greater omentum is the largest peritoneal fold covering the intra-abdominal organs. The greater omentum is highly vascularized, is usually thin and elastic and always contains some fat. As such, the greater omentum has been used in clinical applications, such as intestinal surgery, thoracic esophageal surgery, chronic, nonhealing skin wounds, hernia and pelvic floor repair, bladder repair and the like.

Because the vascular channels of decellularized omentum can serve as tracks for neovascularization, omentum is a desired material for clinical application. The fat within the omentum, however, is difficult to remove using methods and procedures known in the art for decellularizing soft mammalian tissue. Thus, effective use of decellularized omentum for biomatrices, including as a tissue based ECM biomaterial is limited because, in part, the processes and methods for revitalizing tissue described in the art cannot effectively extract the fat from omentum.

The invention concerns methods for extracting fat from omentum, processes for preparing decellularized omentum and medical devices comprising the processed decellularized omentum matrix.

In one aspect, the present invention provides a method for defatting omentum comprising providing an omentum, dehydrating the omentum by contacting the omentum with at least one dehydration solvent, followed by extracting fat from the dehydrated omentum with at least one extraction solvent.

Methods for extracting fat from omentum, which can be applied within decellularization processes described herein as well as in other decellularization are devitalization processes are aspects of the invention. In an embodiment, the fat extraction methods include dehydration of the omentum with one or more dehydration solvents, such as low molecular weight organic solvents such as alcohols, followed by fat extraction with one or more extraction solvents selected from the group consisting of non-polar solvents, polar solvents or combinations thereof. After defatting, the omentum may be rehydrated. In the methods of the invention some of the fat, all of the fat and/or substantially all fat is extracted from the omentum.

Decellularized omentum may be used "as is". Also, the decellularized omentum can used in other applications such as being formulated into films, porous 3D scaffolds, particulates and tubular structures for tissue engineering and regenerative medicine in combination with cells, minced tissue, bioactives and other tissue engineering scaffolds. Omental membrane also has haemostatic properties due to elevated tissue factor (TF) concentrations. Thus, decellularized omentum can be used as hemostats.

Omentum is favored for many applications over other tissue based ECM biomaterial and devices, such as dermis. Omentum has many vascular channels which are preserved in the fat extraction methods and decellularization processes described herein. These vascular channels can serve tracks for neovascularization. The invention provides for effective extraction of fat from the omentum. The ability to effectively extract fat from the omentum enhances the use of omentum as a biomatrix.

All parts and percentages set forth in this Specification and the claims are on a weight-by-weight basis unless otherwise specified.

### BRIEF DESCRIPTION OF THE SEVERAL VIEWS OF THE DRAWINGS

**Fig. 1** is a flowchart of a decellularization process in accordance with an embodiment of the invention.

**Fig. 2** is an image of omentum before and after decellularization.

**Fig. 3** depicts histograms of omentum before and after decellularization.

### DETAILED DESCRIPTION OF THE INVENTION

The present invention concerns processes for preparing an acellular omentum, i.e., devitalized or decellularized omentum, comprising extracellular matrix for implantation into a mammal. The processes generally have the steps of defatting and decellularizing tissue, particularly omentum. An aspect of the invention concerns methods for defatting omentum.

Defatting for purposes of the invention refers to extracting the lipid moiety in the tissue. Decellularization or devitalization for purposes of the invention refers to removing the cellular components of an isolated organ, or a part of an organ, without any significant damage to the extracellular matrix. The terms decellularized or decellurization and devitalized or devitalization shall be used herein interchangeably. Devitalized tissue is essentially free from reproductively and/or metabolically viable cells. Omentum devoid of reproductively viable cells, however, could contain metabolically viable cells that are incapable of increasing the numbers of metabolically viable cells through the normal process of meiosis or mitosis. Also, in the omentum devoid of metabolically viable cells, metabolically dead cells might be visible in histology sections appearing similar to a metabolically live cell when viewed with the use of a microscope; further cellular remnants, including nucleic acids, small molecular weight proteins, lipids, and polysaccharides, while the devitalized tissue retains reproductively non-viable cells and/or metabolically non-viable cells and/or large molecular weight cytoplasmic proteins, such proteins including for example, actin to act as a chemoattractant. Decellularized omentum or devitalized omentum, refers to omentum having all, some or a substantial amount of fat and nuclear and cellular components removed.

Omentum may be harvested from mammalian species, such as human, swine, bovine, goat and the like. Following tissue harvesting, the tissue can be either placed in 0.9% saline for immediate processing or stored for later use, preferably at a temperature of about -20° C to about 80° C.

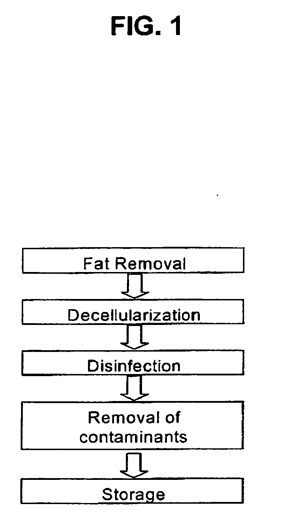

A process for obtaining devitalized omentum in accordance with the invention is set forth in **Fig. 1**. This process has the steps of defatting omentum and decellularizing the defatted omentum with the further, optional, steps of disinfection and removal of contaminants. The devitalized omentum obtained from this process may then be stored until needed for use or may be used immediately after the devitalization process is complete.

The method for defatting the omentum, which may be applied with the decellularization processes described herein or applied with other procedures for obtaining devitalized or decellular omentum, includes dehydration followed by fat extraction and then optionally re-hydrating the defatted omentum. Dehydration involves treating the omentum with one or more dehydration solvents, such one or more treatments of the omentum with a dehydration solvent(s) and/or such solvent(s) in solution with water. The one or more treatments may be sequential steps in the method performed with solutions having different ratios of dehydration solvent(s) to water, such as having gradually reduced amounts of water in the solution for each successive treatment and the final treatment may involve the use of pure solvent, i.e., solvent not in solution with water. After dehydration, the fat may be extracted from the omentum using one or more extraction solvent(s), which may occur in one or more extraction steps.

Low molecular weight organic solvents may be used for the dehydration solvent. In an embodiment, the dehydration solvent is one or more alcohols, such as those selected from the group consisting of methanol, ethanol, isopropanol, propanol and combinations thereof. The dehydration solvent may be in solution with water having about 60% to about 70% alcohol and about 30% to about 40% water.

In an embodiment, the defatting method includes contacting omentum with dehydration solvent for a period of time such as at least about 30 minutes, typically about 30 minutes to about 72 hours. The weight ratio of the omentum to dehydration solvent may be about 1:5 to about 1:100, such as about 1:5 to about 1:50, typically about 1:10 to about 1:25. The omentum may be completely submerged within the dehydration solvent. Dehydration may occur at ambient temperature, although dehydration at temperatures above and below ambient temperature is within the scope of the invention. Embodiments of the defatting method include one or more dehydration treatments in dehydration solvent using the same or different dehydration solvent under the same or different conditions and weight ratios of omentum to dehydration solvent. During each dehydration treatment the solvent may be changed or refreshed periodically.

In an embodiment of the invention, the defatting method involves initially contacting the omentum in a first dehydration solvent having alcohol and water, such as a solution having from about 60% to about 70% alcohol and about 30% to about 40% water. The omentum should be contacted with the first dehydration solvent for at about 30 minutes at ambient temperature, although temperatures below or above ambient temperature are within the scope of the invention. The weight ratio of the omentum to first dehydration solvent may be about 1:5 to about 1:50, such as about 1:10 to about 1:25. In this first treatment, the omentum may be fully submerged in the first dehydration solvent.

After the omentum is treated with the first dehydration solvent, the omentum is contacted with a second dehydration solvent, such as pure alcohol. In this second dehydration step, the omentum may be contacted with the second dehydration solvent for at least about 30 minutes, such as about 30 minutes to about 24 hours. The second dehydration step may be conducted at ambient temperature, although performing this treatment at temperatures below or above ambient temperature is within the scope of the invention. The weight ratio of the omentum to second dehydration solvent may be about 1:5 to about 1:100, such as about 1:10 to about 1:25. The omentum may be fully contacted with the second dehydration solvent.

In a further embodiment, after the second dehydration treatment the omentum may optionally be subjected to one or more further dehydration treatment(s). These further dehydration treatments are conducted under the same conditions and using the same materials and amounts as discussed above with respect to the second dehydration treatment. For example, in an embodiment, after the second dehydration treatment, a third dehydration treatment is conducted in which the omentum from the second dehydration treatment is contacted with, and may be fully submerged, in a third dehydration solvent, such as pure alcohol for at least about 30 minutes, such as about 30 minutes to about 24 hours. The weight ratio of the omentum to third dehydration solvent may be about 1:5 to about 1:100, such as about 1:10 to about 1:25. In another embodiment, after the third dehydration treatment, a fourth dehydration treatment is performed in which the omentum from the third dehydration treatment is contacted with, and may be fully submerged in, a fourth dehydration solvent, such as pure alcohol, for at least about 30 minutes, such as about 30 minutes to about 24 hours. The weight ratio of the omentum to fourth dehydration solvent may be about 1:5 to about 1:100, such as about 1:10 to about 1:25.

After dehydrating, the fat is extracted from the omentum by extraction using one or more extraction solvents. The extraction solvents may be non-polar solvent, polar solvent, such as polar aprotic solvent, or combinations thereof. Examples of non-polar solvents are non-polar organic solvents such as hexane, xylene, benzene, toluene, ethyl acetate and combinations thereof. Polar solvents useful for the extraction solvent include acetone, dioxane, acetonitrile and combinations thereof. In an embodiment, the extraction solvent is selected from acetone, hexane, xylene and combinations thereof. The extraction solvent may have about 50% to about 90% nonpolar solvent, such as hexane, xylene and combinations thereof and about 10% to about 50% polar solvent, such as acetone. In certain embodiments, the extraction solvent may have about 50% to about 70% acetone and about 30% to about 50% hexane.

Fat extraction is conducted in fat extraction steps by contacting the dehydrated omentum with extraction solvents for a period of time. One or more fat extraction steps may be conducted using the same or different extraction solvent(s) under the same or different conditions for the same or different periods of time. In embodiments, the fat extraction may include contacting the dehydrated omentum with one or more extraction solvent(s), or combinations of the extraction solvents, for a period of time of at least about 30 minutes, such as at least about 60 minutes up to about 24 hours, or up to about 48 hours, or more. Further embodiments involve submerging the dehydrated omentum in the extraction solvent. During each extraction step the solvent may be changed or refreshed periodically. In embodiments of the invention, the weight ratio of dehydrated omentum to extraction solvent is about 1:3 to about 1:50.

In an embodiment, the fat extraction is conducted in a plurality of fat extracting steps. Initially, after the last dehydration treatment, such as the second dehydration treatment, third dehydration treatment or fourth dehydration treatment as discussed above, the dehydrated omentum is contacted with a first extraction solvent, such as a polar solvent, such as a polar aprotic solvent, for at least about 30 minutes, typically for about 30 minutes to about 24 hours. The dehydrated omentum may be fully submerged in the first extraction solvent. In an aspect of the invention, the weight ratio of dehydrated omentum to first extraction solvent is about 1:3 to about 1:50, such as about 1:5 to about 1:15. In an embodiment, the first extraction solvent is acetone.

After the first extraction step the dehydrated omentum is contacted with a second extraction solvent, such as a mixture of about 30% to about 50% polar solvent and about 50% to about 70% non-polar solvent for a period to time of at least about 60 minutes, such as about 60 minutes to about 24 hours. In an embodiment, the second extraction solvent has about 30% to about 50% hexane and about 70% to about 50% acetone. The dehydrated omentum may be fully submerged in the second extraction solvent. In an aspect of the invention, the weight ratio of dehydrated omentum to second extraction solvent is about 1:3 to about 1:50, such as about 1:5 to about 1:15.

Next, the dehydrated omentum is subjected to a third extraction step which involves contacting the dehydrated omentum with a third extraction solvent. In an embodiment, the dehydrated omentum is contacted with the third extraction solvent for about 24 hours to about 48 hours and may be submerged in the third extraction solvent for that period of time. The third extraction solvent may have from about 70% to about 90% non-polar solvent and about 10% to about 30% polar solvent, such as a polar aprotic solvent. An embodiment of the invention involves a third extraction solvent having about 70% to about 90% hexane and about 10% to about 30% of acetone. In an aspect of the invention, the weight ratio of dehydrated omentum to third extraction solvent is about 1:3 to about 1:50, such as about 1:5 to about 1:15.

After the fat extraction, the defatted omentum is optionally re-hydrated. The defatted omentum maybe re-hydrated by contacting the defatted omentum with a re-hydration solvent, such as alcohol or a solution of alcohol in water, such as an alcohol solution having from about 60% to about 70% alcohol. Low molecular weight alcohols, such as methanol, ethanol, isopropanol, propanol and combinations thereof may be used. In an embodiment of the invention, re-hydration is conducted in one or more re-hydration treatments, such as two treatments. In an aspect of the invention, the weight ratio of defatted omentum to re-hydration solvent in all of some of the re-hydration treatments is about 1:5 to about 1:100, such as about 1:10 to about 1:25. The defatted omentum may be contacted with the re-hydration solvent for at least 30 minutes, such as about 30 minutes to about 72 hours, and may be fully submerged in the re-hydration solvent.

In an embodiment, re-hydration of the defatted omentum is conducted in two re-hydration treatments. In a first re-hydration treatment, the defatted omentum is contacted with a first re-hydration solvent, typically a low molecular weight alcohol such as ethanol for at least about 30 minutes, such as about 30 minutes to about 72 hours. In an aspect of the invention, the weight ratio of defatted omentum to first re-hydration solvent is about 1:5 to about 1:100, such as about 1:10 to about 1:25. The first re-hydration solvent may be pure alcohol, i.e. not in solution with water. The defatted omentum may be fully submerged in the first re-hydration solvent.

Next, in a second re-hydration treatment the defatted omentum is contacted with a second re-hydration solvent, which may be a low molecular weight alcohol and in particular a low molecular weight alcohol in solution with water, such as about a 60% to 70% solution. In an embodiment, the second re-hydration solvent is ethanol, such as a solution having about 60% to about 70% ethanol with the balance water. The defatted omentum may be contacted with the second re-hydration solvent for at least about 30 minutes, such as about 30 minutes to about 72 hours. In an aspect of the invention, the weight ratio of defatted omentum to second re-hydration solvent is about 1:5 to about 1:100, such as about 1:10 to about 1:25. The defatted omentum may be fully submerged in the second re-hydration solvent.

The defatted omentum is decellularized, and decellularization processes known to one skilled in the art may be applied to decellularize the defatted omentum. In an embodiment, the defatted omentum may be decellularized by solubilization of the nuclear and cytoplasmic components. For example, the defatted omentum may be immersed in a decellularization buffer, such as one having non-ionic detergent and metal salt dissolved in acid for a period of time, typically at least about 30 minutes. Non-ionic detergents useful in the invention include polysorbates, such as TWEEN 80, ethoxylated alcohols, such as TRITON® X-100, and polyethanols, such as HP 40 and IGEPAL CA-630 and combinations thereof. Metal salts that may be used include magnesium chloride, phosphate, acetate and citrate, and combinations thereof and these metal salts are typically dissolved in Tris-HCL. For example, the decellularization buffer may include TRITON ® X-100 (1% w/V) and MgCl₂ (1%) dissolved in 50 mM Tris-HCl (pH 7.2). The defatted omentum is then removed from the decellularization buffer and contacted with an enzyme solution, such as one having endonuclease, such as Benzonase, and the components of the decellularization buffer. In an embodiment, the defatted omentum is spun in the enzyme solution for a period of time, such as at least about 20 hours, typically from about 20 hours to about 48 hours. The defatted omentum is then washed one or more times, such as twice, in a rinsing solution, such as one having acid, metal salt and nonionic detergent. The acid, metal salt and nonionic detergent may be the same as the materials discussed above, including the combination of Tris-HCl, MgCl₂ and TRITON® X-1 00. Subsequently, the omentum is contacted with a cell extracting solution having salts, such as NaCl and EDTA, and non-ionic detergent, such as TRITON® X-100, for a period of time, such as at least about 1 hour, typically from about 1 hour to about 48 hours. Examples of typical decellularization processes are further described in U.S. Patent Numbers 4,776,853 and 4,801,299.

The decellularized omentum may then be disinfected to remove contaminants. In an embodiment, the decellularized omentum is contacted with a disinfection solution for a sufficiently effective period of time to disinfect the decellularized omentum, such as at least about 0.5 hour, typically about 1 hour to about 12 hours. The decellularized omentum may be fully submerged in the disinfection solution. The disinfection solution may have alcohol, or an alcohol in water solution, and may also include acid. The disinfection solution may include one or more of the following ethanol, methanol, isopropanol, propanol, hydrogen peroxide, peracetic acid and combinations thereof. In an embodiment, the disinfection solution has ethanol, such as an 80% ethanol solution, and peracetic acid. Optionally, the decellularized omentum can be washed one or more times with ultrapure water.

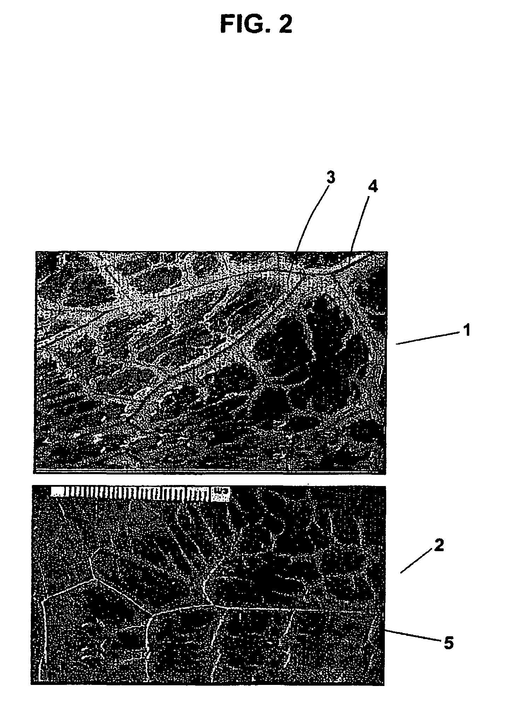

**Fig. 2** shows a comparison of an untreated porcine omentum **1** and a decellularized porcine omentum **2**. The untreated porcine omentum **1** has fat **3** adjacent to vascular tracks **4** whereas in the decellularized porcine omentum, the vascular tracks **5** do not have any or a substantial amount of fat adjacent thereto.

The decellularized omentum may be applied in tissue engineering and regeneration of internal organs, such as kidney, liver, spleen and bladder. The decellularized omentum can also be used for repair and regeneration of skeletal tissues, such as bone, cartilage and tendon. Other uses for the decellularized omentum include soft tissue reinforcement and repair in combination with biocompatible meshes, such as dural grafting, hernia repair, and pelvic floor repair; nerve regeneration, such a tubular structure for peripheral nerve regeneration; tissue augmentation; delivery of cells and bioactives; chronic wound repair; and bone repair. These uses and applications of the decellularized omentum are illustrative of several potential uses and should not be construed as limiting the types of uses and applications for the decellularized omentum prepared by the methods and processes described herein.

The decellularized omentum can be combined with synthetic constructs to make reinforced constructs. For example, the decellularized omentum matrix can be used as a scaffold structure for implantation in a mammalian body, such as scaffold for tissue repair. It can be further enhanced by bioactives, cells, small molecules, minced tissue and cell lysates. The decellularized omentum can be lypophilized with polymers to make 3D foam or heat melted into a film or mesh to name a few additional uses for the decellularized omentum. Further, fibers may be electrostatically spun onto the omentum and used "as is" or with synthetic constructs to make reinforced structures.

In one embodiment, decellularized omentum is formulated into a tubular structure with or without reinforcement. The tubular omentum matrix can be seeded with endothelial cells in which the devitalized omentum serves as a cell attachment scaffold and growth promoting substrate. Endothelial cell seeded onto decellularized omentum has utility as a building block material for vascular reconstruction.

In another embodiment, decellularized omentum can be co-cultured with human kidney derived cells (hKDC) in which the decellularized omentum serves as a cell attachment scaffold and growth promoting substrate. HKDC seeded onto decellularized omentum has utility as a building block material for kidney tissue engineering applications.

In a further embodiment, decellularized omentum can be co-cultured with urothelial cells in which the decellularized omentum serves as a cell attachment scaffold and growth promoting substrate. Urothelial cells seeded onto decellularized omentum have utility as a building block material for bladder reconstruction.

Bioactive agents may be incorporated within and/or applied to the tissue scaffolds, and/or it can be applied to the viable minced tissue that is then incorporated to the scaffolds. Preferably, the bioactive agent is incorporated within, or coated on, the scaffold prior to the addition of viable tissue to the scaffold. The bioactive agent(s) can be selected from among a variety of effectors that, when present at the site of injury, promote healing and/or regeneration of the affected tissue. In addition to being compounds or agents that promote or expedite healing, the effectors may also include compounds or agents that prevent infection (e.g., antimicrobial agents and antibiotics), compounds or agents that reduce inflammation (e.g., anti-inflammatory agents), compounds that prevent or minimize adhesion formation, such as oxidized regenerated cellulose (e.g., INTERCEED® and SURGICEL®, available from Ethicon, Inc., Somerville, New Jersey, USA) compounds or agents that suppress the immune system (e.g., immunosuppressants), and combinations thereof.

By way of non-limiting example, other types of effectors present within an implant of the invention having the decellularized omentum can include heterologous or autologous growth factors, proteins (including matrix proteins), peptides, antibodies, enzymes, platelets, platelet rich plasma, glycoproteins, hormones, cytokines, glycosaminoglycans, nucleic acids, analgesics, viruses, virus particles, cell types and combinations thereof. One or more effectors of the same or different functionality may be incorporated within the implant.

Many different types of heterologous or autologous growth factors known to promote healing and/or regeneration of injured or damaged tissue may be incorporated directly into the scaffold, or alternatively, the scaffold can include a source of growth factors, such as for example, platelets. Bioactive agents, may further include one or more of the following: chemotactic agents; therapeutic agents (e.g., antibiotics, steroidal and non-steroidal analgesics and antiinflammatories, anti-rejection agents such as immunosuppressants and anti-cancer drugs); various proteins (e.g., short term peptides, bone morphogenic proteins, glycoprotein and lipoprotein); cell attachment mediators; biologically active ligands; integrin binding sequence; ligands; various growth and/or differentiation agents and fragments thereof (e.g., epidermal growth factor (EGF), hepatocyte growth factor (HGF), vascular endothelial growth factors (VEGF), fibroblast growth factors (e.g., bFGF), platelet derived growth factors (PDGF), insulin derived growth factor (e.g., IGF-1, IGF-II) and transforming growth factors (e.g., TGF-β I-III), parathyroid hormone, parathyroid hormone related peptide, bone morphogenic proteins (e.g., BMP-2, BMP-4; BMP-6; BMP-12), sonic hedgehog, growth differentiation factors (e.g., GDF5, GDF6, GDF8), recombinant human growth factors (e.g., MP52), cartilage-derived morphogenic proteins (CDMP-1)); small molecules that affect the upregulation of specific growth factors; tenascin-C; hyaluronic acid; chondroitin sulfate; fibronectin; decorin; thromboelastin; thrombin-derived peptides; heparin-binding domains; heparin; heparan sulfate; DNA fragments and DNA plasmids. Suitable effectors likewise include the agonists and antagonists of the agents described above. The growth factor can also include combinations of the growth factors described above. In addition, the growth factor can be autologous growth factor that is supplied by platelets in the blood, which may have various growth factors normally associated with platelets. If other such substances have therapeutic value in the orthopedic field, it is anticipated that at least some of these substances will have use in the invention, and such substances should be included in the meaning of "bioactive agent" and "bioactive agents" unless expressly limited otherwise.

The decellularized omentum can further be formulated into composite scaffolds in a combination with biocompatible synthetic or natural polymers. In one embodiment, the decellularized omentum matrix can be reinforced to enhance its mechanical strength. The reinforcement component can be a 2-D film with or without pores, fiber structure, 3D foam and the like. The reinforced decellularized omentum can be formulated into 2-D films, 3-D matrix and tubular structures with and without 3-D matrix in the lumen for tissue engineering and regenerative medicine in combination with cells, minced tissue, bioactives and other tissue engineering scaffolds.

Varieties of bioabsorbable polymers can be used to the non-woven tissue engineering scaffolds and the mesh having the decellularized omentum according to the invention. Examples of suitable biocompatible, bioabsorbable polymers include polymers selected from the group consisting of aliphatic polyesters, poly (amino acids), copoly (ether- esters), polyalkylenes oxalates, polyamides, tyrosine derived polycarbonates, poly (iminocarbonates), polyorthoesters, polyoxaesters, polyamidoesters, polyoxaesters containing amine groups, poly (anhydrides), polyphosphazenes, biomolecules (i.e., biopolymers such as collagen, elastin, bioabsorbable starches, etc.) and blends thereof. Aliphatic polyesters include, but are not limited to, homopolymers and copolymers of lactide (which includes lactic acid, D-, L- and meso lactide), glycolide (including glycolic acid), epsilon-caprolactone, p-dioxanone (1,4-dioxan-2-one), trimethylene carbonate (1,3- dioxan-2-one), alkyl derivatives of trimethylene carbonate, and polymer blends thereof.

Varieties of biocompatible non-absorbable polymers can also be used to the non-woven tissue engineering scaffolds and the mesh according to the invention. An example is polypropylene mesh, sold under the tradename PROLENE® available from Ethicon, Inc., Somerville, New Jersey, USA.

Varieties of biocompatible natural biopolymers can also be used for the non-woven tissue engineering scaffolds and the mesh having the decellularized omentum in accordance with the invention. Examples of suitable biocompatible, natural polymers include collagen, elastin, hyaluronic acid, laminin, and gelatin.

### EXAMPLES

### Example 1: Solubility Of Swine Fat

Approximately 0.75g swine fat was added into 0.5ml selected solvent. The solubility of the fat was visually observed and the time at which complete solubility occurred was noted. The results are presented in the table. The scale for turbidity is 0 being clear and 5 having high turbidity.

| Solvents | Turbidity | Observation |
|---|---|---|
| acetone | 5 | Fat was liquefied by 120 seconds, but the mixture was highly turbid. |
| hexane | 1 | Fat dissolved completely by 200 seconds. |
| acetone + hexane 50:50 | 2 | Fat dissolved completely by 220 seconds. |
| acetone + hexane 30:70 | 1 | Fat dissolved completely by 150 seconds. |
| acetone + hexane 20:80 | 1 | Fat dissolved completely by 150 seconds. |
| xylene | 0 | Fat dissolved completely by 210 seconds. |

### Example 2: Omentum Decellularization Process

FAT EXTRACTION: Pig omentum was placed in 0.9% saline after harvest. After rinsing in the saline solution 3 times to rinse off blood and other extraneous debris, the omentum was placed in 70% ethanol for 30 minutes. Following the treatment with 70% ethanol, the tissue was dehydrated in 100% ethanol for 30 minutes with two changes into fresh ethanol. The tissue was then transferred to acetone for 180 minutes, using fresh solution every 60 minutes. Subsequently, the tissue was placed in a 50:50 acetone-hexane mixture for 60 minutes, followed by a 20:80 mixture of the same for 24-48 hours (with 3 changes of fresh solution) for fat extraction. The tissue was then transferred to 100% ethanol for 30 minutes and subsequently to 70% ethanol where, if necessary, it could be stored at 4° C until the decellularization process was initiated.

DECELLULARIZATION: The tissue was then immersed in a decellularization buffer having a cocktail of TRITON® X-1 00 (1 % w/V; a nonionic detergent) and MgCl₂ (1%) dissolved in 50 mM Tris-HCl (pH 7.2), for 30 minutes. This was followed by treatment in an enzyme solution having a cocktail of endonuclease (Benzonase; 41.8 U/ml) mixed with the decellularization buffer. The tissue was spun in this solution for 20 hours. The tissue was then washed twice (2 hours each) in a solution having 50 mM Tris-HCl (pH 7.2), 5 mM MgCl₂ and 1% (W/V) TRITON® X-100. The tissue was then placed in a cell extracting solution having 1M NaCl, 20mm EDTA, 0.2% (W/V) TRITON® X-100 pH 7.0 for 1 hr, following which the tissue was washed with ultra pure water (4 times, 5 minutes each).

DISINFECTION: The tissue was transferred to disinfection solution having 80:20 water: ethanol (200 proof) with 0.15% peracetic acid (or acetic acid) for 1 hour after washing in water (4 times, 20 minutes each), the tissue was stored in 70% alcohol at 4° C.

### Example 3: Histological Evaluation

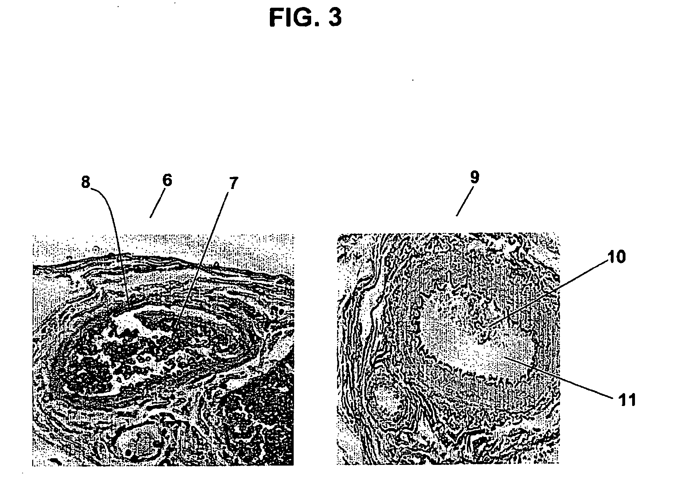

Samples of untreated omentum and decellularized omentum devitalized in accordance Example 2 were fixed in 10% buffered neutral formalin for 2 days. The samples were then processed for routine histology (i.e., washing in water, dehydrating with alcohol, clearing in xylene, embedding in paraffin, sectioning and subsequently processing the slides for staining with hematoxylin and eosin). **Fig. 3** shows the fresh porcine omentum **6** having fat **7** within the matrix **8** whereas in the decellularized omentum **9**, there is much less fat **10** within the matrix **11**.

### Example 4: Growth Of Porcine Urothelial And Human Umbilical Tissue-Derived Cells (hUTCs) On Devitalized Porcine Omentum

Urothelial cells were isolated from porcine bladders. Briefly, porcine bladders were obtained from Farm-to-Pharm (Bridgewater, New Jersey, USA) washed clean with PBS and emptied of urine. Bladders were then cut along their long axis and laid flat with the urothelial side facing upward. They were then covered with 0.25% Trypsin solution for approximately 4 hours. Following this digestion, the urothelial side was scraped with a cell scraper to peel off the urothelium and the digestion liquid was sieved through a 40 micrometer filter. The liquid was then spun down and cells washed with the Keratinocyte Serum Free (KSF) medium (Invitrogen, Carlsbad, California, USA). Cells were plated in T-75 tissue culture flasks using the KSF medium. The medium was changed every 2-3 days.

Human umbilical tissue-derived cells (hUTCs) were isolated as described in United States Patent Application Publication Number US2005/0054098 A1, which is incorporated in its entirety herein by reference, and grown in growth medium (DMEM-low glucose (Gibco, Carlsbad, California, USA), 15% (v/v) fetal bovine serum (Cat. #SH30070.03; Hyclone, Logan, Utah, USA), 0.001% (v/v) betamercaptoethanol (Sigma-Aldrich, St. Louis, Missouri, USA), penicillin/streptomycin (Gibco), in a gelatin-coated T75 flask. Cells were passaged every 3-4 days.

Decellularized omentum prepared in accordance with Example 2 was cut into approximately 2.5 x 2.5 cm squares and placed into ultra low cluster 6-well dishes. Omentum was sterilized by incubation in absolute ethanol for 16 hours (PDS film) or 1 hour (for omentum previously stored in 70% ethanol). Materials were then washed 3 times in 3 mL of PBS (Ca and Mg -free).

Porcine urothelial cells (P1) and hUTCs were harvested by washing with PBS and trypsinization using 4 ml of 0.25% trypsin-EDTA (Gibco). Reaction was stopped by the addition of an equal volume of keratinocyte-SFM (Gibco) for urothelial cells and growth medium for hUTCs. Cells were collected by centrifugation and the cell pellet re-suspended in culture media. Cells were counted using a Guava instrument (Guava Technologies, Hayward, California, USA) and 100,000 cells in 1 ml of media were seeded on the omental membrane in 6-well plates. After incubation for 2 hours at 37° C, an additional 2 ml of media was added to each well. Urothelial cells or hUTCs were seeded 10 days prior to immunofluorescence staining.

On 10th day post-cell seeding, omentum membranes were washed with 3 ml PBS and then fixed with cold 4% (w/v) paraformaldehyde (Sigma-Aldrich) for 10 minutes at room temperature. Immunocytochemistry was performed using antibodies directed against the following epitopes: alpha smooth muscle actin (1:200, Chemicon International cat#: CBL171, Temecula, California, USA) and cytokeratin AE1/AE3 (1:200; Chemicon International cat#: MAB3412).

Cultures were washed with phosphate-buffered saline (PBS) and exposed to a protein blocking solution containing PBS, 4% (v/v) goat serum (Chemicon), and 0.3% (v/v) TRITON® (X-1 00) for 1 hour to access intracellular antigens. Primary antibodies, diluted in blocking solution, were then applied to the cultures for a period of 1 hour at room temperature. Next, primary antibody solutions were removed and cultures washed with PBS prior to application of secondary antibody solutions (1 hour at room temperature) containing block along with goat anti-mouse IgG - FITC conjugate (1:200; Sigma-Aldrich). Cultures were then washed and 10 µM DAPI (Molecular Probes) applied for 10 minutes to visualize cell nuclei.

Following immunostaining, fluorescence was visualized using the appropriate fluorescence filter on an Olympus inverted epi-fluorescent microscope (Olympus, Melville, New York, USA). In all cases, positive staining represented fluorescence signal above control staining where the entire procedure outlined above was followed with the exception of application of a primary antibody solution (no. 1 control). Representative images were captured using a digital color video camera and ImagePro software (Media Cybernetics, Carlsbad, California, USA). For double-stained samples, each image was taken using only one emission filter at a time. Layered montages were then prepared using Adobe Photoshop software (Adobe, San Jose, California, USA).

The results of this experiment indicated that cells of both types grew well on decellularized porcine omental membrane. Furthermore, both cell types established a monolayer and retained robust expression of key markers: keratin for urothelial cells and smooth muscle actin for hUTCs.

### Example 5: Human Kidney Derived Cell Growth On Devitalized Porcine Omentum

Normal human kidneys were obtained from the National Disease Research Interchange (NDRI, Philadelphia, Pennsylvania, USA). The kidney used in this study was obtained from a healthy, 21 year old male. To remove blood and debris, the kidney was washed in Dulbecco's modified Eagles medium (DMEM-low glucose; Invitrogen, Carlsbad, California, USA) or phosphate buffered-saline (PBS; Invitrogen). Tissue was dissected from the cortex region of the kidney. The tissues were then mechanically dissociated in tissue culture plates until the tissue was minced to a fine pulp. The tissue was then transferred to a 50-milliliter conical tube. The tissue was then digested in GMP (good manufacturing practice) enzyme mixtures containing 0.25 units PZ activity/ml collagenase (NB6, N0002779; Serva Electrophoresis GmbH, Heidelberg, Germany), 2.5 units/ml dispase (Dispase II 165 859, Roche Diagnostics Corporation, Indianapolis, Indiana, USA), and 1 unit/mL hyaluronidase (Vitrase, ISTA Pharmaceuticals, Irvine, California, USA). The enzyme mixture was combined with renal epithelial growth medium (REGM) (Cambrex). The conical tubes containing the tissue, medium and digestion enzymes were incubated at 37°C in an orbital shaker at 225 rpm for 1-2 hours.

The digested material was centrifuged at 150 x g for 5 minutes, then the supernatant was aspirated. The pellet was then re-suspended in 20 milliliters REGM. The cell suspension was filtered through a 40-micron nylon BD FALCON Cell strainer (BD Biosciences, San Jose, California, USA). The filtrate was re-suspended in REGM (total volume 50 milliliters) and centrifuged at 150 x g for 5 minutes. The supernatant was aspirated and the cells were re-suspended in 50 milliliters of REGM. This process was repeated twice.

After the final centrifugation, supernatant was aspirated and the cell pellet was re-suspended in 10 milliliters of REGM. The number of viable cells was determined using a Guava instrument (Guava Technologies). Cells were then plated onto gelatin-coated tissue culture flasks and cultured at 37°C under normal atmospheric conditions. Cells were further passaged on non-coated tissue-culture flasks six times with media exchange every 2-3 days. Cells were then cryopreserved under liquid nitrogen.

Omentum membrane decellularized in accordance with Example 2 was cut into several 4 cm² pieces and then submerged in 70% ethanol overnight at 4°C. Individual pieces of omentum membrane were then deposited into wells of a 6 well plate. To remove residual ethanol, the omentum membrane was washed three times with 3 ml of PBS followed by one wash with 3 ml REGM. Passage 6 hKDCs were then thawed and seeded at 10,000 cells/cm² onto the omentum membrane and cultured for four days in REGM. Fresh media was added to the cultures every 2-3 days.

On day four post-cell seeding, omentum membranes were washed with 3 ml PBS and then either stained with 1 um calcein-AM (Molecular Probes) or fixed in 4% paraformaldehyde overnight at room temperature. Calcein-AM stained samples were visualized by fluorescent microscopy and fixed samples were sent to VetPath Services for histological processing and H&E staining.

After four days in culture, hKDCs showed significant attachment to the devitalized omentum. In addition, the cells were viable as evidenced by the intense Calcein-AM staining.

### Example 6: Growth Of Human Umbilical Vein Endothelial Cells (HUVECs) On Devitalized Porcine Omentum

Human umbilical vein endothelial cells (HUVECs, catalog number C2517A) were obtained from Cambrex and were cultured in endothelial growth medium (EGM-2MV, catalog number 3202) according to manufacturer's recommendations which are incorporated herein by reference. For routine passage, cells were washed once with phosphate-buffered saline (PBS, Invitrogen, catalog number 14190) and detached by trypsinization (0.25% trypsin-EDTA, Invitrogen, catalog number 25200-056). Cells were counted using a Guava instrument (Guava Technologies) and seeded at a density of 5000 cells/cm². Cells were routinely passaged every 3-4 days.

Decellularized omentum in accordance with Example 2 was cut into approximately 2.5 x 2.5 cm squares and placed into ultra low cluster 6-well dishes. Omentum was sterilized by incubation in absolute ethanol for 1 hour (omentum previously stored in 70% ethanol). Omentum membrane was washed 3 times in 3 ml of PBS (Ca and Mg -free) before use.

HUVECs were harvested by washing with PBS and trypsinization using 4 ml of 0.25% trypsin-EDTA (Gibco). Reaction was stopped by the addition of an equal volume of EGM-2MV media. Cells were collected by centrifugation and the cell pellet re-suspended in culture media. Cells were counted using a Guava instrument (Guava Technologies) and 100,000 cells in 1 ml of media were seeded on omentum membrane in 6-well plates. After incubation for 2 hours at 37°C, an additional 2 ml of media was added to each well.

HUVECs were grown on omentum membrane for 10 days and processed for DAPI staining. Briefly, omentum membranes were washed with 3 ml PBS and then fixed with cold 4% (w/v) paraformaldehyde (Sigma-Aldrich) for 10 minutes at room temperature. Cultures were washed with PBS and stained with 10 µM DAPI (Molecular Probes) for 15 minutes to visualize cell nuclei. For viability staining, a live/dead staining kit (Molecular Probes) was used. Omentum membranes were washed with 3 ml PBS and then stained with 1 uM calcein-AM and 1 uM propidium iodide for 10 minutes at room temperature.

Cell nuclei (stained with DAPI) and viable cells (stained with calcein-AM) were visualized using the appropriate fluorescence filter on an Olympus inverted epi-fluorescent microscope (Olympus). Representative images were captured using a digital color video camera and ImagePro software (Media Cybernetics).

The results of this study show that human umbilical vein endothelial cells can attach and grow on decellularized porcine omentum membrane and that these cells are viable.

### Example 7: Incorporation of GDF-5 into a composite of non-woven scaffolds and decellularized omentum

Felts made of 90/10 PGA/PLA fibers with an average length of 5cm are placed on both sides of a decellularized omentum prepared in accordance with Example 2. The structure was then needle punched to create the interlock of 90/10 PGA/PLA fibers of the felts with the decellularized omentum. The composites were approximately 1 mm thick with a density of 75 mg/cc. GDF-5 at a concentration of 200 mg/ml in 10 mM HCl was added to the composite scaffold and then lyophilized at -25°C. The GDF-5 loaded composite scoffolds can be potentially used for connective tissue repair, for example cartilage regeneration, bone repair and wound healing.

## Claims

1. A method for defatting omentum comprising providing an omentum, dehydrating the omentum by contacting the omentum with at least one dehydration solvent, followed by extracting fat from the dehydrated omentum with at least one extraction solvent.

2. The method of claim 1 wherein substantially all the fat within the omentum is extracted.

3. The method of Claim 1 or 2 wherein the extraction solvent is selected from the group consisting of non-polar solvent, polar solvent and combinations thereof.

4. The method of Claim 3 wherein the extraction solvent is selected from the group consisting of hexane, xylene, benzene, toluene, ethyl acetate, acetone, dioxane, acetonitrile and combinations thereof.

5. The method of any preceding Claim wherein the dehydration solvent is selected from the group consisting of methanol, ethanol, isopropanol, propanol and combinations thereof.

6. The method of any preceding Claim wherein a weight ratio of the omentum to dehydration solvent is 1:5 to 1:100.

7. The method of any preceding Claim wherein, a weight ratio of dehydrated omentum to extraction solvent is 1:3 to 1:50.

8. The method of any preceding Claim wherein the omentum is dehydrated in one or more treatments.

9. The method of Claim 8 wherein the treatments comprise contacting the omentum with a first dehydration solvent comprising alcohol and water and contacting the omentum with a second dehydration solvent comprising alcohol.

10. The method of Claim 9 wherein the first dehydration solvent comprises from 60% and 70% alcohol and 30% to 40% water and the second dehydration solvent is pure alcohol.

11. The method of Claim 9 or 10 wherein the treatments further comprise contacting the omentum with a third dehydration solvent comprising alcohol and then contacting the omentum with a fourth dehydration solvent comprising alcohol.

12. The method of Claim 11 wherein the third dehydration solvent and second dehydration solvent are pure alcohol.

13. The method of Claim 11 wherein a weight ratio of the omentum to the first dehydration solvent is 1:5 to 1:50, a weight ratio of the omentum to the second dehydration solvent is 1:5 to 1:100, a weight ratio of the omentum to the third dehydration solvent is 1:5 to 1:100 and a weight ratio of the omentum to the fourth dehydration solvent is 1:5 to 1:100.

14. The method of any preceding Claim wherein the fat is extracted from the dehydrated omentum in one or more fat extraction steps.

15. The method of Claim 14 wherein the fat extraction steps comprise contacting the dehydrated omentum with a first extraction solvent which is a polar solvent then contacting the dehydrated omentum with second extraction solvent comprising 30% to 50% polar solvent and 50% to 70% non-polar solvent and then contacting the dehydrated omentum with a third extraction solvent comprising 70% to 90% of non-polar solvent and 10% to 30% polar solvent.

16. The method of Claim 15 wherein the polar solvent is acetone and the non-polar solvent is hexane.

17. The method of Claim 15 wherein a weight ratio of the dehydrated omentum to the first extraction solvent is 1:3 to 1:50, a weight ratio of the dehydrated omentum to the second extraction solvent is 1:3 to 1:50, and a weight ratio of the dehydrated omentum to the third extraction solvent is 1:3 to 1:50.

18. The method of any preceding claim further comprising re-hydrating the omentum after fat extraction.

19. A process according to any preceding claim for preparing a devitalized omentum further comprising the step of decellularizing the defatted omentum.

20. The process of Claim 19 comprising the additional step of disinfecting the decellularized defatted omentum.

21. The process of Claim 19 comprising the additional step of sterilizing the decellularized defatted omentum.

22. A construct for medical purposes comprising a defatted omentum obtainable by a method according to any of claims 1 to 21.

23. A tubular structure for vascular reconstruction comprising decellularized omentum obtainable by a method according to any of claims 1 to 21 seeded with endothelial cells wherein the decellularized omentum comprises a cell attachment scaffold and growth promoting substrate.

24. A scaffold structure for implantation in a mammalian body comprising decellularized omentum obtainable by a method according to any of claims 1 to 21.

25. The scaffold structure of Claim 24 wherein the decellularized omentum is co-cultured with human kidney derived cells and the decellularized omentum comprises a cell attachment scaffold and growth promoting substrate.

26. The scaffold structure of Claim 24 wherein the decellularized omentum is co-cultured with urothelial cells and the decellularized omentum comprises a cell attachment scaffold and growth promoting substrate.

27. The scaffold structure of Claim 24 further comprising bioactive agents.

28. The scaffold structure of Claim 27 wherein the bioactive agent is selected from the group consisting of effectors that promote healing, effectors that promote regeneration of tissue, effectors that promote or expedite healing, compounds or agents that prevent infection, compounds or agents that reduce inflammation, compounds that prevent or minimize adhesion formation, compounds or agents that suppress the immune system and combinations thereof.

29. The scaffold structure of Claim 27 wherein the bioactive agent is selected from the group consisting of heterologous growth factors, autologous growth factors, proteins, peptides, antibodies, enzymes, platelets, platelet rich plasma, glycoproteins, hormones, cytokines, glycosaminoglycans, nucleic acids, analgesics, viruses, virus particles, cell types, therapeutic agents, antiinflammatories, anti- rejection agents, chemotactic agents, growth agents, differentiation agents, growth agent fragments, differentiation agent fragments and combinations thereof.

30. The scaffold structure of Claim 27 wherein the bioactive agent is selected from the group consisting of antibiotics, steroidal analgesics, non-steroidal analgesics, immunosuppressants, anticancer drugs, short term peptides, bone morphogenic proteins, glycoprotein, lipoprotein, cell attachment mediators, biologically active ligands, integrin binding sequence, ligands, epidermal growth factor, hepatocyte growth factor, vascular endothelial growth factors, fibroblast growth factors, platelet derived growth factors, insulin derived growth factor, transforming growth factors, parathyroid hormone, parathyroid hormone related peptide, sonic hedgehog, growth differentiation factors, recombinant human growth factors, cartilage-derived morphogenic proteins, small molecules, small molecules that affect the upregulation of specific growth factors, tenascin-C, hyaluronic acid, chondroitin sulfate, fibronectin, decorin, thromboelastin, thrombin-dehved peptides, heparin-binding domains, heparin, heparan sulfate, DNA fragments, DNA plasmids, and combinations thereof.

31. The scaffold structure of Claim 24 comprising a composite of the decellularized omentum and a polymer selected from the group consisting of biocompatible synthetic polymers and natural polymers.

32. The scaffold structure of Claim 31 wherein the biocompatible polymer is selected from the group consisting of polypropylene, aliphatic polyesters, poly (amino acids), copoly (ether-esters), polyalkylenes oxalates, polyamides, tyrosine derived polycarbonates, poly (iminocarbonates), polyorthoesters, polyoxaesters, polyamidoesters, polyoxaesters containing amine groups, poly (anhydrides), polyphosphazenes, biomolecules (i.e., biopolymers such as collagen, elastin, bioabsorbable starches, etc.) and blends thereof.

33. The scaffold structure of Claim 31 wherein the natural polymer is selected from the group consisting of collagen, elastin, hyaluronic acid, laminin and gelatin.

## Patentansprüche

1. Verfahren zum Entfetten von Omentum, umfassend das Bereitstellen eines Omentums, Dehydratisieren des Omentums durch Zusammenbringen des Omentums mit mindestens einem Dehydratisierungs-Lösungsmittel, und danach Extraktion von Fett aus dem dehydratisierten Omentum mit mindestens einem Extraktions-Lösungsmittel.

2. Verfahren nach Anspruch 1, wobei im Wesentlichen das gesamte Fett in dem Omentum extrahiert wird.

3. Verfahren nach Anspruch 1 oder 2, wobei das Extraktions-Lösungsmittel aus der aus unpolarem Lösungsmittel, polarem Lösungsmittel und Kombinationen davon bestehenden Gruppe ausgewählt ist.

4. Verfahren nach Anspruch 3, wobei das Extraktions-Lösungsmittel aus der aus Hexan, Xylol, Benzol, Toluol, Ethylacetat, Aceton, Dioxan, Acetonitril, und Kombinationen davon bestehenden Gruppe ausgewählt ist.

5. Verfahren nach irgendeinem vorhergehenden Anspruch, wobei das Dehydratisierungs-Lösungsmittel aus der aus Methanol, Ethanol, Isopropanol, Propanol und Kombinationen davon bestehenden Gruppe ausgewählt ist.

6. Verfahren nach irgendeinem vorhergehenden Anspruch, wobei das Gewichtsverhältnis des Omentums zum Dehydratisierungs-Lösungsmittel von 1:5 bis 1:100 reicht.

7. Verfahren nach irgendeinem vorhergehenden Anspruch, wobei das Gewichtsverhältnis des dehydratisierten Omentums zum Extraktions-Lösungsmittel von 1:3 bis 1:50 reicht.

8. Verfahren nach irgendeinem vorhergehenden Anspruch, wobei das Omentum in einer oder mehreren Behandlungen dehydratisiert wird.

9. Verfahren nach Anspruch 8, wobei die Behandlungen das Zusammenbringen des Omentums mit einem ersten Dehydratisierungs-Lösungsmittel, das Alkohol und Wasser umfasst, und das Zusammenbringen des Omentums mit einem zweiten Dehydratisierungs-Lösungsmittel, welches Alkohol umfasst, umfassen.

10. Verfahren nach Anspruch 9, wobei das erste Dehydratisierungs-Lösungsmittel 60% bis 70% Alkohol und 30% bis 40% Wasser umfasst, und das zweite Dehydratisierungs-Lösungsmittel reiner Alkohol ist.

11. Verfahren nach Anspruch 9 oder 10, wobei die Behandlungen zudem das Zusammenbringen des Omentums mit einem dritten Dehydratisierungs-Lösungsmittel, das Alkohol umfasst, und dann das Zusammenbringen des Omentums mit einem vierten Dehydratisierungs-Lösungsmittel, das Alkohol umfasst, umfassen.

12. Verfahren nach Anspruch 11, wobei das dritte Dehydratisierungs-Lösungsmittel und das zweite Dehydratisierungs-Lösungsmittel reiner Alkohol sind.

13. Verfahren nach Anspruch 11, wobei das Gewichtsverhältnis des Omentums zum ersten Dehydratisierungs-Lösungsmittel 1:5 bis 1:50 ist, das Gewichtsverhältnis des Omentums zum zweiten Dehydratisierungs-Lösungsmittel 1:5 bis 1:100 ist, das Gewichtsverhältnis des Omentums zum dritten Dehydratisierungs-Lösungsmittel 1:5 bis 1:100 ist und das das Gewichtsverhältnis des Omentums zum vierten Dehydratisierungs-Lösungsmittel 1:5 bis 1:100 ist.

14. Verfahren nach irgendeinem vorhergehenden Anspruch, wobei das Fett aus dem dehydratisierten Omentum in einem oder mehreren Fettextraktionsschritten extrahiert wird.

15. Verfahren nach Anspruch 14, wobei die Fettextraktionsschritte das Zusammenbringen des dehydratisierten Omentums mit einem ersten Extraktions-Lösungsmittel, bei dem es sich um ein polares Lösungsmittel handelt, dann das Zusammenbringen des dehydratisierten Omentums mit einem zweiten Extraktions-Lösungsmittel, das 30% bis 50% polares Lösungsmittel und 50% bis 70% unpolares Lösungsmittel umfasst, und dann das Zusammenbringen des dehydratisierten Omentums mit einem dritten Extraktions-Lösungsmittel, das 70% bis 90% unpolares Lösungsmittel und 10% bis 30% polares Lösungsmittel umfasst, umfassen.

16. Verfahren nach Anspruch 15, wobei das polare Lösungsmittel Aceton ist, und das unpolare Lösungsmittel Hexan ist.

17. Verfahren nach Anspruch 15, wobei das Gewichtsverhältnis des dehydratisierten Omentums zum ersten Extraktions-Lösungsmittel 1:3 bis 1:50 ist, das Gewichtsverhältnis des dehydratisierten Omentums zum zweiten Extraktions-Lösungsmittel 1:3 bis 1:50 ist und das Gewichtsverhältnis des dehydratisierten Omentums zum dritten Extraktions-Lösungsmittel 1:3 bis 1:50 ist.

18. Verfahren nach irgendeinem vorhergehenden Anspruch, zudem umfassend das Rehydratisieren des Omentums nach der Fettextraktion.

19. Prozess nach irgendeinem vorhergehenden Anspruch zum Herstellen eines devitalisierten Omentums, zudem umfassend den Schritt Dezellularisieren des entfetteten Omentums.

20. Prozess nach Anspruch 19, umfassend den zusätzlichen Schritt Desinfizieren des dezellularisierten entfetteten Omentums.

21. Prozess nach Anspruch 19, umfassend den zusätzlichen Schritt Sterilisieren des dezellularisierten entfetteten Omentums.

22. Konstrukt für medizinische Zwecke, umfassend ein entfettetes Omentum, das sich durch ein Verfahren nach irgendeinem der Ansprüche 1 bis 21 erhalten lässt.

23. Röhrenstruktur zur Gefäßrekonstruktion, umfassend dezellularisiertes Omentum, das sich erhalten lässt durch ein Verfahren nach irgendeinem der Ansprüche 1 bis 21, das mit Endothelzellen beimpft wurde, wobei das dezellularisierte Omentum ein Zellbindungsgerüst und wachstumsförderndes Substrat umfasst.

24. Gerüststruktur zum Anwenden in einem Säugetierkörper, umfassend dezellularisiertes Omentum, das sich durch ein Verfahren nach irgendeinem der Ansprüche 1 bis 21 erhalten lässt.

25. Gerüststruktur nach Anspruch 24, wobei das dezellularisierte Omentum zusammen mit Zellen cokultiviert wird, die aus menschlicher Niere stammen, und das dezellularisierte Omentum ein Zellbindungsgerüst und wachstumsförderndes Substrat umfasst.

26. Gerüststruktur nach Anspruch 24, wobei das dezellularisierte Omentum zusammen mit Urothel-Zellen cokultiviert wird, und das dezellularisierte Omentum ein Zellbindungsgerüst und wachstumsförderndes Substrat umfasst.

27. Gerüststruktur nach Anspruch 24, zudem umfassend biologisch aktive Mittel.

28. Gerüststruktur nach Anspruch 27, wobei das biologisch aktive Mittel aus der aus Effektoren, die die Heilung fördern, Effektoren, die die Regeneration von Gewebe fördern, Effektoren, die die Heilung fördern oder beschleunigen, Verbindungen oder Mitteln, die die Infektion verhindern, Verbindungen oder Mitteln, die die Entzündung reduzieren, Verbindungen, die die Adhäsionsbildung verhindern oder minimieren, Verbindungen oder Mitteln, die das Immunsystem unterdrücken und Kombinationen davon bestehenden Gruppe ausgewählt ist.

29. Gerüststruktur nach Anspruch 27, wobei das biologisch aktive Mittel aus der aus heterologen Wachstumsfaktoren, autologen Wachstumsfaktoren, Proteinen, Peptiden, Antikörpern, Enzymen, Blutplättchen, blutplättchenreichem Plasma, Glycoproteinen, Hormonen, Cytokinen, Glycosaminoglycanen, Nukleinsäuren, Analgetika, Viren, Viruspartikeln, Zelltypen, Therapeutika, entzündungshemmenden Mitteln, Mitteln, die die Abstoßung verhindern, chemotaktischen Mitteln, Wachstumsmitteln, Differenzierungsmitteln, Wachstumsmittelfragmenten, Differenzierungsmittelfragmenten und Kombinationen davon bestehenden Gruppe ausgewählt ist.

30. Gerüststruktur nach Anspruch 27 wobei das biologisch aktive Mittel aus der aus Antibiotika,
steroidalen Analgetika, nicht-steroidalen Analgetika, Immunsuppressiva, Anti-Krebs-Arzneimitteln, Kurzzeitpeptiden, Knochenmorphogenproteinen, Glycoprotein, Lipoprotein, Zellbindungsvermittlern, biologisch aktiven Liganden, Integrinbindungssequenz, Liganden, epidermalem Wachstumsfaktor, Hepatocyten-Wachstumsfaktor, Gefäßendothel-Wachstumsfaktoren, Fibroblasten-Wachstumsfaktoren, von Blutplättchen stammenden Wachstumsfaktoren, von Insulin stammendem Wachstumsfaktor, transformierenden Wachstumsfaktoren, Parathyroidhormon, Parathyroidhormon-verwandtem Peptid, Sonic Hedgehog, Wachstumsdifferenzierungsfaktoren, rekombinanten menschlichen Wachstumsfaktoren, aus Knorpel stammenden Morphogenproteinen, kleinen Molekülen, kleinen Molekülen, die die Aufwärtsregulation spezifischer Wachstumsfaktoren beeinträchtigen, Tenascin-C, Hyaluronsäure, Chondroitinsulfat, Fibronectin, Decorin, Thromboelastin, von Thrombin stammenden Peptiden, Heparin bindenden Domänen, Heparin, Heparansulfat, DNA-Fragmenten, DNA-Plasmiden, und Kombinationen davon bestehenden Gruppe ausgewählt ist.

31. Gerüststruktur nach Anspruch 24, umfassend einen Verbundstoff des dezellularisierten Omentums und eines Polymers, das aus der aus biokompatiblen synthetischen Polymeren und natürlichen Polymeren bestehenden Gruppe ausgewählt ist.

32. Gerüststruktur nach Anspruch 31 wobei das biokompatible Polymer aus der aus Polypropylen, aliphatischen Polyestern, Poly(aminosäuren), Copoly(etherestern), Polyalkylenen Oxalaten, Polyamiden, von Tyrosin stammenden Polycarbonaten, Poly(iminocarbonaten), Polyorthoestern, Polyoxaestern, Polyamidoestern, Polyoxaestern mit Amingruppen, Poly(anhydriden), Polyphosphazenen, Biomolekülen (d.h. Biopolymeren wie Kollagen, Elastin, bioabsorbierbaren Stärken usw.) und Gemischen davon bestehenden Gruppe ausgewählt ist.

33. Gerüststruktur nach Anspruch 31 wobei das natürliche Polymer aus der aus Collagen, Elastin, Hyaluronsäure, Laminin und Gelatine bestehenden Gruppe ausgewählt ist.

## Revendications

1. Méthode de dégraissage de l'épiploon comprenant l'obtention d'un épiploon, la déshydratation de l'épiploon par mise en contact de l'épiploon avec au moins un solvant déshydratant, puis l'extraction des graisses de l'épiploon déshydraté avec au moins un solvant d'extraction.

2. Méthode selon la revendication 1, **caractérisée en ce que** sensiblement l'ensemble des graisses dans l'épiploon sont extraites.

3. Méthode selon la revendication 1 ou 2, **caractérisée en ce que** le solvant d'extraction est choisi dans le groupe constitué par un solvant non polaire, un solvant polaire et des combinaisons de ceux-ci.

4. Méthode selon la revendication 3, **caractérisée en ce que** le solvant d'extraction est choisi dans le groupe constitué par l'hexane, le xylène, le benzène, le toluène, l'acétate d'éthyle, l'acétone, le dioxane, l'acétonitrile et des combinaisons de ceux-ci.

5. Méthode selon l'une quelconque des revendications précédentes, **caractérisée en ce que** le solvant de déshydratation est choisi dans le groupe constitué par le méthanol, l'éthanol, l'isopropanol, le propanol et des combinaisons de ceux-ci.

6. Méthode selon l'une quelconque des revendications précédentes, **caractérisée en ce que** le rapport en poids entre l'épiploon et le solvant déshydratant est de 1:5 à 1:100.

7. Méthode selon l'une quelconque des revendications précédentes, **caractérisée en ce que** le rapport en poids entre l'épiploon déshydraté et le solvant d'extraction est de 1:3 à 1:50.

8. Méthode selon l'une quelconque des revendications précédentes, **caractérisée en ce que** l'épiploon est déshydraté en un ou plusieurs traitements.

9. Méthode selon l'une quelconque des revendications précédentes, **caractérisée en ce que** les traitements comprennent la mise en contact de l'épiploon avec un premier solvant déshydratant comprenant de l'alcool et de l'eau et la mise en contact de l'épiploon avec un deuxième solvant déshydratant comprenant de l'alcool.

10. Méthode selon la revendication 9, **caractérisée en ce que** le premier solvant déshydratant comprend de 60 % à 70 % d'alcool et de 30 % à 40 % d'eau et le deuxième solvant déshydratant est de l'alcool pur.

11. Méthode selon la revendication 9 ou 10, **caractérisée en ce que** les traitements comprennent en outre la mise en contact de l'épiploon avec un troisième solvant déshydratant comprenant de l'alcool, puis la mise en contact de l'épiploon avec un quatrième solvant déshydratant comprenant de l'alcool.

12. Méthode selon la revendication 11, **caractérisée en ce que** le troisième solvant déshydratant et le deuxième solvant déshydratant sont de l'alcool pur.

13. Méthode selon la revendication 11, **caractérisée en ce que** le rapport en poids entre l'épiploon et le premier solvant déshydratant est de 1:5 à 1:50, le rapport en poids entre l'épiploon et le deuxième solvant déshydratant est de 1:5 à 1:100, le rapport en poids entre l'épiploon et le troisième solvant déshydratant est de 1:5 à 1:100 et le rapport en poids entre l'épiploon et le quatrième solvant déshydratant est de 1:5 à 1:100.

14. Méthode selon l'une quelconque des revendications précédentes, **caractérisée en ce que** les graisses sont extraites de l'épiploon déshydraté en une ou plusieurs étapes d'extraction de graisse.

15. Méthode selon la revendication 14, **caractérisée en ce que** les étapes d'extraction de graisse comprennent la mise en contact de l'épiploon déshydraté avec un premier solvant d'extraction qui est un solvant polaire, puis la mise en contact de l'épiploon déshydraté avec un deuxième solvant d'extraction comprenant de 30 % à 50 % de solvant polaire et de 50 % à 70 % de solvant non polaire, puis la mise en contact de l'épiploon déshydraté avec un troisième solvant d'extraction comprenant de 70 % à 90 % de solvant non polaire et de 10 % à 30 % de solvant polaire.

16. Méthode selon la revendication 15, **caractérisée en ce que** le solvant polaire est l'acétone et le solvant non polaire est l'hexane.

17. Méthode selon la revendication 15, **caractérisée en ce que** le rapport en poids entre l'épiploon déshydraté et le premier solvant d'extraction est de 1:3 à 1:50, le rapport en poids entre l'épiploon déshydraté et le deuxième solvant d'extraction est de 1:3 à 1:50 et le rapport en poids entre l'épiploon déshydraté et le troisième solvant d'extraction est de 1:3 à 1:50.

18. Méthode selon l'une quelconque des revendications précédentes, comprenant en outre la réhydratation de l'épiploon après l'extraction de graisse.

19. Procédé selon l'une quelconque des revendications précédentes, de préparation d'un épiploon dévitalisé, comprenant en outre l'étape de décellularisation de l'épiploon dégraissé.

20. Procédé selon la revendication 19, comprenant l'étape supplémentaire de désinfection de l'épiploon dégraissé décellularisé.

21. Procédé selon la revendication 19, comprenant l'étape supplémentaire de stérilisation de l'épiploon dégraissé décellularisé.

22. Construction à des fins médicales comprenant un épiploon dégraissé susceptible d'âtre obtenu par une méthode selon l'une quelconque des revendications 1 à 21.

23. Structure tubulaire pour la reconstruction vasculaire, comprenant de l'épiploon décellularisé susceptible d'être obtenu par une méthode selon l'une quelconque des revendications 1 à 21 ensemencé avec des cellules endothéliales, l'épiploon décellularisé comprenant un échafaudage de liaison de cellules et un substrat favorisant la croissance.

24. Structure d'échafaudage pour implantation dans un corps de mammifère comprenant l'épiploon décellularisé susceptible d'être obtenu par une méthode selon l'une quelconque des revendications 1 à 21.

25. Structure d'échafaudage selon la revendication 24, **caractérisée en ce que** l'épiploon décellularisé est mis en co-culture avec des cellules dérivées de rein humain et l'épiploon décellularisé comprend un échafaudage de liaison de cellules et un substrat favorisant la croissance.

26. Structure d'échafaudage selon la revendication 24, **caractérisée en ce que** l'épiploon décellularisé est mis en co-culture avec des cellules urothéliales et l'épiploon décellularisé comprend un échafaudage de liaison de cellules et un substrat favorisant la croissance.

27. Structure d'échafaudage selon la revendication 24, comprenant en outre des agents bioactifs.

28. Structure d'échafaudage selon la revendication 27, **caractérisée en ce que** l'agent bioactif est choisi dans le groupe constitué par les effecteurs qui favorisent la cicatrisation, les effecteurs qui favorisent la régénération des tissus, les effecteurs qui favorisent ou accélèrent la cicatrisation, les composés ou les agents qui empêchent l'infection, les composés ou les agents qui réduisent l'inflammation, les composés qui empêchent ou minimisent la formation d'adhésions, les composés ou les agents qui assurent la suppression du système immunitaire et des combinaisons de ceux-ci.

29. Structure d'échafaudage selon la revendication 27, **caractérisée en ce que** l'agent bioactif est choisi dans le groupe constitué par les facteurs de croissance hétérologues, les facteurs de croissance autologues, les protéines, les peptides, les anticorps, les enzymes, les plaquettes, le plasma riche en plaquettes, les glycoprotéines, les hormones, les cytokines, les glycosaminoglycanes, les acides nucléiques, les analgésiques, les virus, les particules virales, les types cellulaires, les agents thérapeutiques, les agents anti-inflammatoires, les agents anti-rejet, les agents chimiotactiques, les agents de croissance, les agents de différentiation, les fragments d'agents de croissance, les fragments d'agents de différentiation, et des combinaisons de ceux-ci.

30. Structure d'échafaudage selon la revendication 27, **caractérisée en ce que** l'agent bioactif est choisi dans le groupe constitué par les antibiotiques, les analgésiques stéroïdiens, les analgésiques non stéroïdiens, les immunosuppresseurs, les agents anti-cancéreux, les peptides court terme, les protéines morphogéniques osseuses, la glycoprotéine, la lipoprotéine, les médiateurs de liaison de cellules, les ligands biologiquement actifs, la séquence de liaison de l'intégrine, les ligands, le facteur de croissance épidermique, le facteur de croissance des hépatocytes, les facteurs de croissance de l'endothélium vasculaire, les facteurs de croissance des fibroblastes, les facteurs de croissance dérivés des plaquettes, le facteur de croissance dérivé de l'insuline, les facteurs de croissance transformants, l'hormone parathyroïdienne, le peptide apparenté à l'hormone parathyroïdienne, la protéine sonic hedgehog, les facteurs de différentiation de croissance, les facteurs de croissance recombinants humains, les protéines morphogéniques dérivées du cartilage, les petites molécules, les petites molécules qui affectent la régulation à la hausse de facteurs de croissance spécifiques, la ténascine-C, l'acide hyaluronique, le sulfate de chondroïtine, la fibronectine, la décorine, la thromboélastine, les peptides dérivés de la thrombine, les domaines de liaison d'héparine, l'héparine, le sulfate d'héparane, les fragments d'ADN, les plasmides d'ADN, et des combinaisons de ceux-ci.

31. Structure d'échafaudage selon la revendication 24, comprenant un composite de l'épiploon décellularisé et un polymère choisi dans le groupe constitué par les polymères de synthèse biocompatibles et les polymères naturels.

32. Structure d'échafaudage selon la revendication 31, **caractérisée en ce que** le polymère biocompatible est choisi dans le groupe constitué par le polypropylène, les polyesters aliphatiques, les poly(aminoacides), les co-poly(éther-esters), les poly(oxalates d'alkylène), les polyamides, les polycarbonates dérivés de la tyrosine, les poly(iminocarbonates), les poly(ortho esters), les poly(oxaesters), les poly(amidoesters), les poly(oxaesters) contenant des groupements amine, les poly(anhydrides), les polyphosphazènes, les biomolécules (c'est-à-dire les biopolymères tels que le collagène, l'élastine, les amidons bioabsorbables, et similaires) et des mélanges de ceux-ci.

33. Structure d'échafaudage selon la revendication 31, **caractérisée en ce que** le polymère naturel est choisi dans le groupe constitué par le collagène, l'élastine, l'acide hyaluronique, la laminine et la gélatine.
